(19) Europäisches Patentamt
European Patent Office
Office européen des brevets

(11) **EP 3 066 981 A1**

(12) **EUROPEAN PATENT APPLICATION**
published in accordance with Art. 153(4) EPC

(43) Date of publication:
**14.09.2016 Bulletin 2016/37**

(51) Int Cl.:
***A61B 5/05*** (2006.01)

(21) Application number: **14858675.3**

(22) Date of filing: **04.11.2014**

(86) International application number:
**PCT/ES2014/070822**

(87) International publication number:
**WO 2015/063360 (07.05.2015 Gazette 2015/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **04.11.2013 ES 201301062 P**

(71) Applicants:
• **Universidad de Sevilla**
**41013 Sevilla (ES)**
• **Ciber-BBN**
**41013 Sevilla (ES)**

(72) Inventors:
• **ROA ROMERO, Laura Maria**
**E-41013 Sevilla (ES)**
• **REINA TOSINA, Luis Javier**
**E-41013 Sevilla (ES)**
• **NARANJO HERNANDEZ, David**
**E-41013 Sevilla (ES)**
• **ESTUDILLO VALDERRAMA, Miguel Angel**
**E-41013 Sevilla (ES)**

(74) Representative: **Pons**
**Glorieta Ruben Dario 4**
**28010 Madrid (ES)**

(54) **INTELLIGENT BIOIMPEDANCE SENSOR FOR BIOMEDICAL APPLICATIONS**

(57)     The invention relates to an intelligent bioimpedance sensor for biomedical applications, which can carry out bioimpedance measurements in a plurality of configurable frequencies, process data in order to obtain the modulus and the phase of the bioimpedance (or real and imaginary part of the bioimpedance) in each of the frequencies, and wirelessly transmit the results of the processing, configured by means of a device that is in contact with the biological means to be measured by means of a series of electrodes in such a way that the device injects electrical current, via said electrodes, into the biological means, in the different frequencies, and measures the tension generated by the circulation of said current based on the joint operation of various subsystems.

Figure 2

## Description

The present invention, as expressed in the title of this descriptive document, concerns a portable sensing device capable of performing bioimpedance measurements at multiple configurable frequencies, processing the data to obtain the modulus and phase of the bioimpedance (or real and imaginary part of the bioimpedance) in each of the frequencies, and wirelessly transmit the processing results.

It has application in the area of Information and Communications Technology (ICT) in the context of biomedical engineering and medical technology, for the development of portable electronic devices for continuous monitoring of physiological variables of people and their health status.

## BACKGROUND ON THE STATE OF THE ART

The bioimpedance measurement technique is based on the injection into the human body or in a tissue of an alternating electric current of very small intensity, well below the threshold of perception. The electric current produces a voltage drop, the higher, the greater the electrical impedance of the tissue. This technique was introduced in 1930, when Atzler and Lehman found that fluid changes in the chest cavity as a result of the blood pumping by the heart also produced changes in the thoracic impedance [1]. Holzer et al. were the first to apply an AC signal to prevent electrode polarization problems in bioimpedance measurements [2]. In 1966, in collaboration with the Apollo program, the first device to monitor hemodynamic parameters was developed, opening the way to the development of impedance cardiography for the estimation of stroke volume [1]. This principle is the basis of the impedance cardiograph, which has also been used for several decades for the estimation of the cardiac output by Kubicek equation [3].

Thereafter, bioimpedance has been applied in the development of new instruments and medical diagnostic devices. In 1978 Webster and Henderson attempted to reproduce the X-ray tomography techniques, using low frequency electrical signals [4]. But it was not until the 80s, when the University of Sheffield developed the basis of what is understood today as electrical impedance tomography, thanks to which, by measuring the electric potential on the body surface, images related to the impedance distribution inside a body can be obtained [5], [6].

Given that the tissue impedance changes according to the physiological state, this technique has also been used to monitor the viability of transplanted organs [7], to know the state of hydration of the skin or diagnose cutaneous pathologies [8], [9], and even as a method for the noninvasive measurement of blood glucose level [10]-[12]. Furthermore, bioimpedance has been used in the clinical laboratory as a tool for cell measurements (Coulter counter, measurements of hematocrit or cell cultures monitoring) [1], [13]-[17] and the detection of substances in Lab-on-a-Chip [18].

One of the most important applications of bioimpedance is the study of body composition, which is of great clinical utility in different areas: nephrology [1], [19]-[23], nutrition [24], [25], obstetrics [26], gastroenterology [27], postoperative monitoring [28], HIV-infected patients [29], with a deficit of growth hormone [30], obesity [31], [32] or in critical care [33], [34]. In 1963, Tomasset performed the first estimations of total body water by bioimpedance measurements of the whole body using an alternating current of fixed-frequency [35].

Since then, bioimpedance measurements have been used extensively in many patents, which present methods and devices for both the quantification of body composition, the estimation of liquid volumes and the anatomical location of masses (muscles, fat, water) [36]-[44] as well as for other applications such as the estimation of blood pressure [45], [46], stroke volume [47], cardiac output [48]-[52], respiratory rate and heart rate [52]-[55], blood glucose level [56], [57] or tissue monitoring [47], among others.

There are several patents that explain the internal components that comprise the devices and their operation to obtain the bioimpedance signal. Patents showing more detail merely are limited to describe a comprehensive overview of the main elements used by the patented device to perform the measurement. In a generalized manner, the used scheme comprises the following elements: a sensing stage composed by several electrodes together with the electronics responsible for acquiring the bioimpedance signal, which typically include filter stages, amplifiers and analog/digital (A/D) and digital/analog (D/A) converters; a processor or computing element; a memory for storing relevant data; and, only in some cases, a communication stage for sending the processed data outside. However, the description depth of these modules at internal level is often insufficient, and in particular, the analysis of the detection electronics and the signal conditioning is often scarce. For example, this is the case of (US7917202) patent [40], whose main contribution is a refined model that includes the contributions of intracellular tissue to allow a more accurate measurement at two or more frequencies. However, for the measurement of the bioimpedance signal, the authors refer to specialized medical instrumentation (from Xitron Technologies manufacturer), without going into details. (US6615077) patent [41], which includes a method for determining the body dry weight of a patient by segmental measurements based on bioelectrical impedance analysis, also uses a solution of the same manufacturer for the acquisition of bioimpedance data. Again, (US7945317) patent [58], which describes an improved multi-frequency method to perform a bioimpedance analysis of a body segment, suggests the utilization of a commercial solution for the application of the current and the detection of the voltage. The same applies to (US20110275922) patent [59], which indicates that the data processing can be performed by this equipment or on-line using an external computer. (US20060122540) patent [60] provides a method for determining the hydration status of patients on peritoneal dialysis and hemodialysis, and describes the modules used by the device to calculate the circumference of a body segment, based on a digital signal processor (DSP). However, like the others, the use of commercial medical instrumentation is recommended for the measurement system.

Furthermore, a set of patents present specific contributions to the global scheme previously posed, aimed at the improvement of some of the elements of the device electronics involved in the measurement process. For example, (US20130046165) patent [61] describes a bioimpedance capacitive sensor that includes an ad-hoc signal preprocessor coupled to the sensor. Moreover, in one of its preferred embodiments, the sensing circuit measures the relative impedance employing one or more Wheatstone bridges. (US20060004300) patent [62] presents a method to estimate the bioimpedance at multiple frequencies by a LFSR circuit (Linear Feedback Shift Register), which produces a pseudo-random sequence that feeds the D/A converter. (US7457660) patent [63] includes a mechanism for removing errors in bioimpedance measurements, based on the separation of bioimpedance value of other sources of error using measurements of two similar body sections. (US20050012414) patent [64] presents a device especially designed to provide a second power supply for electronic devices of floating type, and in this way the device meets the requirements of medical safety for bioimpedance measurement. (US20040171963) patent [65] presents a more accurate method for the estimation of body composition that corrects a bioelectrical impedance parameter, which reduces the load for the distribution of extracellular fluid. To that purpose, an exchange unit of electrodes is used, which allows the use of up to 8 electrode configurations. (US7974691) patent [66] refers to (US6076015) [67] patent, which uses microamperometric pulses of 20 microseconds and its voltage response for the measurements, repeated at intervals of 50 milliseconds. The impedance circuit of (US6370424) patent [68] uses a balanced biphasic current pulse that avoids the transfer of net charge to the electrodes, and thus the corrosion and deposition of the electrodes is reduced to gain a better biocompatibility. (US20100081960) patent [69] presents a bioimpedance sensor that stands out over other approaches based on the current injection into the tissue because of the use of optical methods whose precision is higher, but also their associated electronics. (EP2567657A1) patent [70] includes in one of its claims, as the main contribution, the grouping of a reference unit and one or more measurement units connected together in a bus including one or more electrical conductors. On the other hand, (US20070142733) patent [71] presents a method of signal separation based on a specific algorithm which is performed partially in an implantable device, in order to reduce interferences. (US7706872) patent [72] describes a method for the measurement of bioelectrical impedance characterized by a periodic excitation signal in the form of square pulses, applied to the input of the object to be measured, whose output is connected to a synchronous detector. The patent notes that the use of rectangular signals ensures that the device has a simple design and low power consumption, and describes a method for increasing the accuracy of bioimpedance measurements through a set of functional blocks.

Although some of them have been commented, there is a broader set of relevant features to include in the design of the patented bioimpedance devices, such as portability, low cost, low-power consumption, ability to communicate with the environment, and personalization to the user, among others. The use of these devices opens the way to the development of new emerging paradigms of health care, such as e-Health and m-Health. But again, few inventions have provided these considerations. For example, (US7930021) patent [42] details a small size apparatus for the measurement of body composition by means of electrodes disposed in the hilt of the apparatus, which must be held by both hands. The advantage of this apparatus over others is its size, which allows it to be carried by the subject. In one of the claims of (US20050101875) patent [73], which is intended in a general way to the monitoring of cardiac vital signs, a monitor/sensor is presented (preferably portable, low cost and with limited battery), which may be disposable. In addition, the monitor electronics can include a wired or wireless link to transmit data. (US20130046165) patent [61] mentioned above also presents a disposable low-cost capacitive sensor. (US6532384) patent [74] presents a battery-powered handheld device with buttons and screen. (US7783344) patent [43] includes in one of its embodiments the measurement of segmental impedance, with the ability to wireless transmission to a remote device. (US5876353) patent [54] presents an impedance monitor to detect edema by the evaluation of the respiratory rate, which communicates wirelessly with a device worn on the wrist and this in turn with a remote fixed device through a telephone line. Other complementary approach is the use of the processing power and the connectivity of commercial portable devices such as PDAs (US6790178) [75], to perform the processing of multiple physiological variables (including bioimpedance). In this case, the sensors are attached to the PDA or have the ability to transfer data to a memory card that can then be inserted into the PDA. In (US20120035432) patent [44], an interesting device is analyzed, which can communicate with the healthcare provider in the same room or remotely and wirelessly via an intermediate device, establishing a bidirectional communication system. Furthermore, the (US20120035432) document [44] addresses another important design issue: the personalization of bioimpedance measurements to the specific features of a patient. However, no patent has the ability to adapt to the users in real time without their intervention.

The smart bioimpedance sensor proposed in this document has a number of features described in the form of innovative features that none of the revised patents fully meet, thanks mainly to the modular design used that comprises six subsystems: sensing, processing, communications, data storage, timing and power. First, one of the most important advantages of the patent is related to the improved accuracy of the bioimpedance measurement, as well as addition of advanced measurement capabilities, primarily from the perspective of two subsystems: sensing and processing. Regarding the sensing subsystem, it is novel the difference of signals previous to the multiplication and the possibilities of the two modules of internal gain control. Such operations allow the hardware reuse in two different and complementary detection schemes: one that results in a novel expansion and modification of the generic scheme of signals in quadrature, and another completely novel based on the successive approximation to the bioimpedance value. Furthermore, the sensing subsystem includes an optimization procedure of the dynamic range of signal values to adaptively provide the best possible estimation of the bioimpedance measurement. Specifically, when the resolution of the A/D converter is insufficient to maintain the sampling error below 1% of the measured signal, one of the modules of the processing subsystem is responsible for increasing the measurement accuracy. Other novelties are an internal multiplexer that allows the hardware reuse for the measurement of the bioimpedance module, and an external multiplexer for the measurement in different sections of the biological medium.

Regarding the processing subsystem, most of the revised patents employ a single frequency (typically 50 kHz). In contrast, the device here proposed is able to develop an on-demand analysis in both single-frequency and multi-frequency modes, by means of novel approaches to the bioimpedance calculation carried out by up to five processing modules. This analysis is performed from several quasi-simultaneous multi-frequency measurements in different sections of the body in the range between 1 kHz and 5 MHz, which can be remotely expanded at both low and high frequencies. The value and number of frequencies are also parameters that can be remotely configured, thus representing another novel aspect of this patent. In addition, the processing subsystem enables advanced monitoring capabilities of both modulus and phase at a single frequency, which has not been reported so far. The frequency value of the impedance can be remotely configured and adapted in real-time to adapt to the particular characteristics of the user, the application, or in order to increase the sensitivity of the device. Another advantage of this invention is related to the sensing subsystem, which allows the independence of the bioimpedance measurement with respect to the electrode type thanks to a coupling module. In this way, the number of application possibilities is considerably increased as well as the non-dependence on existing medical instrumentation shown by other patents.

Second, the communication subsystem presented in the modular design of the patent allows performing several functionalities. On the one hand, it is able to update the processing modules, remotely and seamlessly to the user, i.e. without technical assistance, in order to perform a personalized measurement by the adaptation of the processing algorithms and their parameters to the subject under test. Moreover, its connectivity capabilities are increased compared to other patents thanks to the flexibility of the design of the communication subsystem. Thus, this subsystem allows a bidirectional communication between the monitored subject and the service provider (data sending to the supplier, in the up-link, and command sending and remote configuration to the user, in the down-link), both in real time and delayed operation. For example, in the preferred embodiment, IEEE 802.15.1 and 802.15.4 standards are detailed as possible options for implementation, which support several wireless communications technologies currently commercially available (Bluetooth, Zigbee), as well as other technologies designed to reduce the power consumption (Bluetooth Low Energy). This advantage, which has not been provided by previous patents, together with its connectivity features, makes this device ideal for the development of the aforementioned new health paradigms (m-Health, e-Health).

Third, and generally to all the subsystems that comprise the invention, the patent here presented achieves several objectives thanks to its accessible and sustainable design philosophy. Firstly, by reducing the size of the device, so that an effective portability is allowed in such a way that the user is able to perform the measurement anytime anywhere, without the need for technical assistance. Secondly, the sustainability of the invention is achieved by optimizing and simplifying the measurement electronics and the number of iterations required, leading to lower implementation costs and energy-saving of the smart sensor. Specifically, in the processing subsystem there is a pre-configured protocol that defines the processing modules that can be activated, the activation sequence and the operating parameters in order to achieve the autonomous operation of the smart sensor. This protocol can be remotely and wirelessly re-configured via commands. Furthermore, there is a global signal that is responsible for the activation of the processing subsystem module to prevent excessive power consumption when a bioimpedance measurement is not being performed. On the other hand, the optimization of the design of the sensing subsystem has been possible thanks to a novel approach based on a modification of the generic detection scheme of signal in quadrature used in other inventions, which incorporates new modules, functions and interconnection schemes that make this system completely original. In particular, the scheme here employed uses a single multiplier, reducing costs compared with other devices, and avoiding errors due to possible differences between components. As an example of the low cost related to the smart sensor invention, the possibility of implementing the processing subsystem with a basic 8-bit microprocessor operating at 4MHz is described in the preferred embodiment, in contrast with other patents that propose more expensive technologies such as computers or PDAs. Thus, the present invention is aligned with the objective of sustainable design necessary for health technology innovation.

**EP 3 066 981 A1**

References cited in the Background of the State of Art.-

[1] L. M. Roa, D. Naranjo, L. J. Reina, A. Lara, J. A. Milan, M. A. Estudillo, and J. S. Oliva, "Applications of bioimpedance to end stage renal disease (esrd)," Studies in Computational Intelligence, vol. 404, pp. 689-769, 2013.

[2] G. Eninia and P. Ondzuls, "A portable rheograph for clinical studies." Biull. Eksp. Biol. Med., vol. 52, pp. 105-107, 1961.

[3] W. Kubicek, J. Karnegis, R. Patterson, D. Witsoe, and R. Mattson, "Development and evaluation of an impedance cardiac output system."Aerospace medicine, vol. 37, no. 12, pp. 1208-1212, 1966.

[4] R. Henderson and J. Webster, "An impedance camera for spatially specific measurements of the thorax," IEEE Transactions on Biomedical Engineering, vol. 25, no. 3, pp. 250-254, 1978.

[5] A. Liston, R. Bayford, and D. Holder, "The effect of layers in imaging brain function using electrical impedance tomograghy," Physiological Measurement, vol. 25, no. 1, pp. 143-158,2004.

[6] N. Li, H. Xu, Z. Zhou, J. Xin, Z. Sun, and X. Xu, "Reconfigurable bioimpedance emulation system for electrical impedance tomography system validation," IEEE Transactions on Biomedical Circuits and Systems, vol. 7, no. 4, pp. 460-468, 2013.

[7] J. Edd and B. Rubinsky, "Assessment of the viability of transplant organs with 3d electrical impedance tomography," Annual International Conference of the IEEE Engineering in Medicine and Biology, 2005.

[8] P. Aberg, I. Nicancer, and S. Ollmar, "Minimally invasive electrical impedance spectroscopy of skin exemplified by skin cancer assessments," Annual International Conference of the IEEE Engineering in Medicine and Biology, 2003.

[9] M. Fiedler, L.-C. Gerhardt, S. Derler, G. Bischofberger, C. Hrny, and T. Munzer, "Assessment of biophysical skin properties at different body sites in hospitalized old patients: Results of a pilot study," Gerontology, vol. 58, no. 6, pp. 513-517, 2012.

[10] C. Amaral and B. Wolf, "Effects of glucose in blood and skin impedance spectroscopy," IEEE AFRICON Conference, 2007.

[11] A. Tura, A. Maran, and G. Pacini, "Non-invasive glucose monitoring: Assessment of technologies and devices according to quantitative criteria," Diabetes Research and Clinical Practice, vol. 77, no. 1, pp. 16-40, 2007.

[12] R. Lumbroso, N. Naas, L. Beitel, M. Lawrence, and M. Trifiro, "Novel bioimpedance sensor for glucose recognition," Conference Proceedings of the International Symposium on Signals, Systems and Electronics, 2007.

[13] N. Piacentini, D. Demarchi, P. Civera, and M. Knaflitz, "Blood cell counting by means of impedance measurements in a microsystem device," Proceedings of the 30th Annual International Conference of the IEEE Engineering in Medicine and Biology Society, 2008.

[14] G. Pop, L. Bisschops, B. Iliev, P. Struijk, J. Hoeven, and C. Hoedemaekers, "On-line blood viscosity monitoring in vivo with a central venous catheter, using electrical impedance technique," Biosensors and Bioelectronics, vol. 41, no. 1, pp. 595-601, 2013.

[15] S. Zheng, M. Nandra, and Y.-C. Tai, "Human blood cell sensing with platinum black electroplated impedance sensor," Proceedings of the 2nd IEEE International Conference on Nano/Micro Engineered and Molecular Systems, 2007.

[16] D. Trebbels, D. Hradetzky, and R. Zengerle, "Capacitive on-line hematocrit sensor design based on impedance spectroscopy for use in hemodialysis machines," Annual International Conference of the IEEE Engineering in Medicine and Biology Society. IEEE Engineering in Medicine and Biology Society, vol. 2009, pp. 1208-1211, 2009.

[17] D. Dziong, P. Bagnaninchi, R. Kearney, and M. Tabrizian, "Nondestructive online in vitro monitoring of pre-osteoblast cell proliferation within microporous polymer scaffolds," IEEE Transactions on Nanobioscience, vol. 6, no. 3, pp. 249-258, 2007.

[18] V. Srinivasaraghavan, J. Strobl, and M. Agah, "Bioimpedance rise in response to histone deacetylase inhibitor is a marker of mammary cancer cells within a mixed culture of normal breast cells," Lab on a Chip - Miniaturisation for Chemistry and Biology, vol. 12, no. 24, pp. 5168-5179, 2012.

[19] I. Laegreid, A. Bye, K. Aasarod, and M. Jordhoy, "Nutritional problems, overhydration and the association with quality of life in elderly dialysis patients," International urology and nephrology, vol. 44, no. 6, pp. 1885-1892, 2012.

[20] S. Sipahi, E. Hur, S. Demirtas, I. Kocayigit, D. Bozkurt, A. Tamer, H. Gunduz, and S. Duman, "Body composition monitor measurement technique for the detection of volume status in peritoneal dialysis patients: The effect of abdominal fullness," International Urology and Nephrology, vol. 43, no. 4, pp. 1195-1199, 2011.

[21] L. Liu, F. Zhu, J. G Raimann, S. Thijssen, M. Sipahioglu, G. Wystrychowski, T. Kitzler, C. Tetta, P. Wabel, P. Kotanko, and N. Levin, "Determination of fluid status in haemodialysis patients with whole body and calf bioimpedance techniques," Nephrology, vol. 17, no. 2, pp. 131-140, 2012.

[22] H.-R. Chua, L. Xiang, P.-Y. Chow, H. Xu, L. Shen, E. Lee, and B.-W. Teo, "Quantifying acute changes in volume and nutritional status during haemodialysis using bioimpedance analysis," Nephrology, vol. 17, no. 8, pp. 695-702, 2012.

[23] S. Parmentier, H. Schirutschke, B. Schmitt, J. Schewe, K. Herbrig, F. Pistrosch, and J. Passauer, "Influence of peritoneal dialysis solution on measurements of fluid status by bioimpedance spectroscopy," International Urology and Nephrology, vol. 45, no. 1, pp. 229-232, 2013.

[24] S. Poloni, I. Schweigert Perry, V. D'Almeida, and I. Schwartz, "Does phase angle correlate with hyperhomocysteinemia? a study of patients with classical homocystinuria," Clinical Nutrition, vol. 32, no. 3, pp. 479-480, 2013.

[25] J. Wilson, B. Strauss, B. Fan, F. Duewer, and J. Shepherd, "Improved 4-compartment body-composition model for a clinically accessible measure of total body protein1-3," American Journal of Clinical Nutrition, vol. 97, no. 3, pp. 497-504, 2013.

[26] F. Samani, R. Jabbary, and O. Mashrabi, "Study on uterine artery blood flow in myomatous uterus," Life Science Journal, vol. 9, no. 4, pp. 583-586, 2012.

[27] P. Lundin, M. Karpefors, K. Carlsson, M. Hansen, and M. Ruth, "Bioimpedance spectroscopy: A new tool to assess early esophageal changes linked to gastroesophageal reflux disease?" Diseases of the Esophagus, vol. 24, no. 7, pp. 462-469, 2011.

[28] V. Sharma, A. Singh, B. Kansara, and A. Karlekar, "Comparison of transthoracic electrical bioimpedance cardiac output measurement with thermodilution method in post coronary artery bypass graft patients," Annals of Cardiac Anaesthesia, vol. 14, no. 2, pp. 104-110,2011.

[29] A. Sharma, F. Tian, M. Yin, M. Keller, M. Cohen, and P. Tien, "Association of regional body composition with bone mineral density in HIV-infected and HIV-uninfected momen: Women's interagency HIV study," Journal of Acquired Immune Deficiency Syndromes, vol. 61, no. 4, pp. 469-476, 2012.

[30] C. Di Somma, L. Angrisani, F. Rota, M. Savanelli, T. Cascella, A. Belfiore, F. Orio, G. Lombardi, A. Colao, and S. Savastano, "Gh and igf-i deficiency are associated with reduced loss of fat mass after laparoscopic-adjustable silicone gastric banding," Clinical Endocrinology, vol. 69, no. 3, pp. 393-399, 2008.

[31] E. Sillanpä, S. Cheng, K. Hakkinen, T. Finni, S. Walker, A. Pesola, J. Ahtiainen, L. Stenroth, H. Selänne, and S. Sipilä, "Body composition in 18- to 88-year-old adults-comparison of multifrequency bioimpedance and dual-energy x-ray absorptiometry," Obesity, 22 (1), pp. 101-109, 2014.

[32] L. Donini, E. Poggiogalle, S. Migliaccio, A. Aversa, and A. Pinto, "Body composition in sarcopenic obesity: systematic review of the literature," Mediterranean Journal of Nutrition and Metabolism, pp. 1-8, 2013.

[33] P. Marik, "Noninvasive cardiac output monitors: A state-of the-art review," Journal of Cardiothoracic and Vascular Anesthesia, vol. 27, no. 1, pp. 121-134, 2013.

[34] M. Savalle, F. Gillaizeau, G. Maruani, E. Puymirat, F. Bellenfant, P. Houillier, J.-Y. Fagon, and C. Faisy, "Assessment of body cell mass at bedside in critically ill patients," American Journal of Physiology - Endocrinology and Metabolism, vol. 303, no. 3, pp. 13 E389-E396,2012.

[35] R. Baumgartner, S. Heymsfield, S. Lichtman, J. Wang, and R. Pierson Jr., "Body composition in elderly people: Effect of criterion estimates on predictive equations," American Journal of Clinical Nutrition, vol. 53, no. 6, pp. 1345-1353, 1991.

[36] M. Kraemer and P. Chamney, "Determining the hydration status of a patient," Patent US 7 133 716, Nov. 7, 2006.

[37] P. Chamney and P.Wabel, "Method and a device for determining the hydration and/or nutrition status of a patient," Patent US 2008/0 086 058, Apr. 10, 2008.

[38] N. Levin and F. Zhu, "Bioimpedance methods and apparatus," Patent US 2007/0 027 402, Feb. 1, 2007.

[39] J. Rosell, P. Riu, R. Pallas, J. Elvira, and R. Bragos, "Apparatus and procedure for measuring volumes and global and segmental corporal composition in human beings," Patent US 6 151 523, Nov. 21, 2000.

[40] P. Chammney and P. Wabel, "Method and a device for determining the hydration and/or nutrition status of a patient," Patent US 7 917 202, Mar. 29, 2011.

[41] F. Zhu and N. Levin, "Device and method for monitoring and controlling physiologic parameters of a dialysis patient using segmental bioimpedance," Patent US 6 615 077, Sep. 2, 2003.

[42] T. Ashida, T. Koike, and K. Andachi, "Body composition measuring apparatus," Patent US 7 930 021, Apr. 19, 2011.

[43] R. Lackey, D. Drinan, and E. C.F., "Hydration monitoring," Patent US 7 783 344, Aug. 24,2010.

[44] R. Katra, N. Chakravarthy, and I. Libbus, "Medical device and methods of monitoring a patient with renal dysfunction," Patent US 2012/0 035 432, Feb. 9, 2012.

[45] B. Sramek, "Noninvasive continuous mean arterial blood pressure monitor," Patent US 4 807 638, Feb. 28, 1989.

[46] B. Sramek, "System for therapeutic management of hemodynamic state of patient," Patent US 5 103 828, Apr. 14, 1992.

[47] D. Berstein, "Apparatus for determination of stroke volume using the brachial artery," Patent US 7 261 697, Aug. 28, 2007.

[48] B. Sramek, "Esophageal electrode array for electrical bioimpedance measurement," Patent US 4 836 214, Jun.

6, 1989.

[49] W. Reining, "Impedance cardiograph apparatus and method," Patent US 5 505 209, Apr. 9, 1996.

[50] A. Shmulewitz, "Apparatus and method of bioelectrical impedance analysis of blood flow," Patent US 5 782 774, Jul. 21, 1998.

[51] S. Mortazavi, E. Park, G. Bornzin, J. Florio, J. Sholder, and R. Weyant, "Methods and apparatus for measuring impedance in the body," Patent US 6 044 294, Mar. 28, 2000.

[52] S. Schookin, V. Zubenko, K. Beliaev, A. Morozov, and W. Yong, "Non-invasive monitoring of hemodynamic parameters using impedance cardiography," Patent US 6 161 038" Dec. 12, 2000.

[53] V. Vysin and B. Sramek, "Diastolic clamp for bioimpedance measuring device," Patent US 4 870 578, Sep. 26, 1989.

[54] K. Riff, "Impedance monitor for discerning edema through evaluation of respiratory rate," Patent US 5 876 353, Mar. 2, 1999.

[55] D. Prutchi, "Non-invasive cardiorespiratory monitor with synchronized bioimpedance sensing," Patent US 6 022 322, Feb. 8, 2000.

[56] M. Fuller, D. Deamer, M. Iverson, and A. Koshy, "Radio frequency spectral analysis for in-vitro or in-vivo environments," Patent US 5 792 668, Aug. 11, 1998.

[57] S. Monfre, K. Hazen, T. Ruchti, T. Blank, and J. Henderson, "Method and apparatus using alternative site glucose determinations to calibrate and maintain noninvasive and implantable analyzers," Patent US 6 998 247, Feb. 14, 2006.

[58] N. Levin and F. Zhu, "Bioimpedance methods and apparatus," Patent US 7 945 317, May. 17, 2011.

[59] N. Levin and F. Zhu, "Bioimpedance methods and apparatus," Patent US 2011/0 275 922, Nov. 10, 2011.

[60] F. Zhu and N. Levin, "Device and method for the determination of dry weight by continuous measurement of resistance and calculation of circumference in a body segment using segmental bioimpedance analysis," Patent US 2006/0 122 540, Jun. 8, 2006.

[61] D. Cassidy and H. S. Ng, "System for a disposable capacitive bioimpedance sensor," Patent US 2013/0 046 165, Feb. 21, 2013.

[62] J. Kennedy, "Multifrequency bioimpedance determination," Patent US 2006/0 004 300, Jan. 5, 2006.

[63] K. Smith and J. Ironstone, "Eliminating interface artifact errors in bioimpedance measurements," Patent US 7 457 660, Nov. 25, 2008.

[64] E. Gersing, "Method and apparatus for isolated transformation of a first voltage into a second voltage for measurement of electrical bioimpedances or bioconductances," Patent US 2005/0 012 414, Jan. 20, 2005.

[65] K. Takehara, "Body composition estimation method and body composition measuring apparatus," Patent US 2004/0 171 963, Sep. 2, 2004.

[66] Y. Zhang, "Method and apparatus for controlling cardiac resynchronization therapy using cardiac impedance," Patent US 7 974 691, Jul. 5, 2011.

[67] J. Hartley, M. Cohen, N. Stessman, S. Reedstrom, S. Check, and J. Nelson, "Rate adaptive cardiac rhythm management device using transthoracic impedance," Patent US 6 076 015, Jun. 13,2000.

[68] David Prutchi, "Non-invasive cardiorespiratory monitor with synchronized bioimpedance sensing," Patent US 6 370 424, Jun. 12, 2001.

[69] E. McKenna, "Bioimpedance system and sensor and technique for using the same," Patent US 2010/0 081 960, Feb. 9, 2010.

[70] O. Chételat, "Synchronization and communication bus for biopotential and bioimpedance measurement systems," Patent EP 2 567 657, Mar. 13, 2013.

[71] J. Hatlestad, M. Brockway, Y. Dalal, and L. Moon, "Bio-impedance sensor and sensing method," Patent US 2007/0 142 733, Jun. 21, 2007.

[72] M. Min, A. Kink, R. Land, and T. Parve, "Method and device for measurement of electrical bioimpedance," Patent US 7 706 872, Apr. 27, 2010.

[73] H. Semler and P. Benz, "Non-invasive body composition monitor, system and method," Patent US 2005/0 101 875, May. 12, 2005.

[74] Y. Fukuda, "Bioelectrical impedance measuring method and body composition measuring apparatus," Patent US 6 532 384, Mar. 11, 2003.

[75] J. Mault, N. Johnson, and J. Sanderson, "Physiological monitor and associated computation, display and communication unit," Patent US 6 790 178, Sep. 14, 2004.

## EXPLANATION OF THE INVENTION

By way of explanation of the "Bioimpedance Smart Sensor for Biomedical Applications", this is configured by means of a device that is in contact with the biological medium through an electrode array. This injects an electric current into the biological medium at different frequencies and measures the voltage drop according to the following subsystems:

1. Sensing subsystem: includes the necessary hardware for bioimpedance measurement. This subsystem generates an alternating current of certain amplitude which is injected into the human body through two electrodes (distal electrodes). By means of two other electrodes in the current path (proximal electrodes), the sensing subsystem performs a measurement of the voltage drop caused by the current flow in the biological medium, which can be a part of the body (one arm, leg, trunk, arm + trunk + leg, etc.), an organ or a tissue, and even a liquid with biological substances in suspension. The sensing subsystem operates with these signals to generate other processed signals which allow the estimation of the magnitude and phase of the impedance.

2. Processing subsystem: this subsystem integrates the hardware, software and firmware elements (*program usually recorded in an read-only-memory, which establishes the lowest level logic that controls the electronic circuits* of a *device*) of the bioimpedance smart sensor, used to estimate the magnitude and phase of the bioimpedance (the result can also be the real and imaginary parts of the bioimpedance) in each frequency. These frequencies can be remotely configured by sending a command. The processing subsystem is also responsible for the proper activation and configuration of the sensing subsystem's modules whenever a new bioimpedance measurement is performed. This reduces the power consumption of the bioimpedance smart sensor, leading to low-power operation modes. In addition, each measurement represents a sequence of operations in the sensing subsystem, which are in turn managed by the processing subsystem. Bioimpedance measurements can be activated locally on the bioimpedance smart sensor by activating a switch. They can also be remotely activated by sending a command. In addition, an assortment of time instants for the measurements can also be remotely configured.

3. Communication subsystem: the subsystem integrates the hardware, software and firmware elements to manage the development of the wireless communications of the bioimpedance smart sensor. The communications are bidirectional in order to allow the transmission of the results of the processing subsystem (modulus and phase bioimpedance values at different frequencies, or real and imaginary parts of the bioimpedance values) in the up-link and in the down-link, the remote configuration of the smart sensor through a set of commands.

4. Data Storage Subsystem: This subsystem is responsible for the proper storage of data used by the bioimpedance smart sensor (measured values, auxiliary variables, processing results, settings...).

5. Timing Subsystem: this is responsible for the maintenance of a real-time timing system and the allocation of each measurement to the temporal instant when they were performed for registration and monitoring. This subsystem subsequently reports to the processing subsystem about the preconfigured instants for performing operations.

6. Power subsystem: this is responsible for providing the supply voltages necessary for the proper operation of other subsystems.

A) <u>Sensing Subsystem.-</u>

The sensing subsystem is comprised by the following functional modules:

1. Signal generation module ($M_1$).
This module generates a constant-amplitude ($A_1$) sinusoidal voltage signal ($S_1$). The frequency ($f_1$) of the ($S_1$) signal can be configured to perform real-time bioimpedance measurements in different frequencies. These frequencies can be remotely set through a command in order to take any value between 1 kHz and 5 MHz, though this range can be extended at low and high frequencies depending on the application. The number of frequency points is therefore a configurable parameter, being also possible to perform a single-frequency analysis.

2. Injection signal amplification module ($M_2$).
A gain amplifier ($A_2$) is applied to the ($S_1$) signal to generate the ($S_2$) signal. The function of this module is to decouple the current injection module ($M_1$) and to adapt the voltage levels at the input of ($M_3$) module.

3. Voltage-current conversion module ($M_3$).
A transconductance amplifier that converts the voltage signal ($S_2$) at the output of the module of injection signal amplification into a current signal ($S_3$) with the same frequency ($f_1$), which will be injected into the body section, tissue or biological medium where the bioimpedance measurement is performed. The amplitude of the injected current ($A_I$) has a preset constant value in order to comply with international safety standards. Furthermore, this current amplitude is independent of the impedance of the biological medium, the electrode impedance and the frequency at which the measurement is performed. Two electrodes (distal electrodes) inject the current generated in the biological medium to be analyzed. The section introducing the coupling module ($M_{15}$) describes in more detail the characteristics of these electrodes.

4. Signal detection module ($M_4$).
The flow of the electric-current signal ($S_3$) through the biological medium generates a voltage signal ($S_B$) between the two other electrodes (proximal electrodes). The frequency of this signal is the same as that of ($S_1$), but the

amplitude ($A_B$) of the voltage produced and the phase shift ($\varphi_B$) with respect to signal ($S_7$) depend on tissue characteristics. ($M_4$) module is an instrumentation amplifier which amplifies with a specific gain ($A_4$) the voltage detected by the proximal electrodes, generating a signal ($S_4$). The input impedance of the instrumentation amplifier is very high such that that the voltage drop in the proximal electrodes can be considered negligible, thus obtaining a true measure of the voltage level in the biological medium.

5. Detected signal amplification module ($M_5$).

Amplifier applied to the signal ($S_4$) to generate the signal ($S_5$). The gain ($A_5$) of the amplifier can be configured in real time in order to adaptively optimize the accuracy of the bioimpedance measurement system.

6. Internal signal generation module ($M_6$).

The function of this module is to generate an internal sinusoidal voltage signal ($S_6$) with the same amplitude value ($A_1$) and with the same frequency ($f_1$) of the signal, but with a phase difference ($\varphi_6$) configurable in real time.

7. Internal signal amplification module ($M_7$).

Amplifier with gain ($A_7$) applied to the signal ($S_6$) to generate the signal ($S_7$). The function of this module is to decouple the module ($M_6$) from the sensing subsystem while adapting the voltage-level of the sinusoidal signal into the appropriate levels.

8. Selection module ($M_8$).

Multiplexer whose output signal ($S_8$) can be configured in order to correspond with the signal ($S_7$) in the A position or signal ($S_4$) in the B position.

9. Subtraction module ($M_9$).

This module generates the signal ($S_9$) resulting from subtracting signal ($S_5$) to signal ($S_8$).

10. Multiplication module ($M_{10}$).

This module generates the signal ($S_{10}$) as a result of the multiplication of the signal ($S_8$) and the signal ($S_9$). The resulting signal ($S_{10}$) will be formed by the sum of a sinusoidal signal with ($2*f_1$) frequency and a DC level dependent on the phase shift between the two signals and their amplitudes.

11. Filtering module ($M_{11}$).

This module generates the signal ($S_{11}$) as a result of the low pass filtering of the signal ($S_{10}$), which removes the ($2*f_1$) sinusoidal frequency component. The filter cutoff frequency is low enough to keep the ripple of the signal below 1% over the DC level in all operating frequencies.

12. Amplifier module of the filtered signal ($M_{12}$).

Signal amplifier applied to the signal ($S_{11}$) to generate the signal ($S_{12}$). The gain of this amplifier ($A_{12}$) can be configured in real time in order to adaptively optimize the accuracy of the bioimpedance measurement system.

13. Analog-to-Digital conversion module ($M_{13}$).

This module is responsible for converting the analog signal ($S_{12}$) into digital signals with which the processing subsystem can operate. When the resolution of the Analog-to-Digital converter is not high enough in order to maintain the sampling error below 1% of the measured signal, the ($MP_2$) processing module of the processing subsystem will increase the measurement accuracy.

14. Multiplexing module ($M_{14}$).

When this module is implemented, it allows measurements in different parts of the body to be automatically made. For this purpose, the electrodes are positioned at the different parts of the body to be measured for both current injection and voltage detection phases. This module is responsible for driving the current signal ($S_3$) into the injection electrodes and lead signal ($S_4$) from the detection electrodes.

15. Coupling module ($M_{15}$).

It is composed by the electrodes of the sensing system for both current injection (distal electrodes) and detection voltage (proximal electrodes), and by the cables connecting these electrodes with the multiplexing module ($M_{14}$). Generally, the module ($M_{15}$) is arranged externally to the rest of the modules of the bioimpedance smart sensor, which are integrated with the rest of subsystems within the same housing. The cables are permanently attached to the module ($M_{14}$) or through one or more connectors in the housing. The cable length can be adapted to the specific sensor application. In addition, they are shielded to protect against external interferences. The electrodes may be wet or dry, of different shapes and sizes, with or without adhesive depending on the application type. The measurement technique employed, with an injected current amplitude independent of the electrode impedance, and an instrumentation amplifier that cancels the effects of the sensing electrodes impedance, allows the system to be independent of the characteristics of the electrodes. The only constraints to be considered are, on the one hand, that the contact impedance between the electrode and the biological environment is low enough so that the voltage generated by the injected current is within the operating range of the device, and on the other hand that its dimensions are such that the current density is below 1 mA/cm$^2$ (for non- in-vivo measurements this density can be increased). The cable connections with the electrodes may be fixed or being established through metal clamps, depending on the application. For in-vivo measurements, cables and electrodes can also be arranged on clothes, or being part of them (electrotextiles), which can be set on the part of the body to be measured. In this case, the smart sensor housing would be built on the same clothes or would be prepared to be coupled thereto, thus forming a portable device. Finally, another possibility is that the electrodes form an integral part of the smart sensor housing itself. In addition to the aforementioned four-electrode configuration (two electrodes for current injection and two voltage sensing electrodes), other configurations are also possible with three or two electrodes, in which one or both injection electrodes respectively correspond with one or both sensing electrodes. In these other configurations, however, the impedance of the electrodes will inevitably affect the impedance measurement.

B) Processing Subsystem.-

The processing subsystem is formed by five processing modules ($MP_1$), ($MP_2$), ($MP_3$), ($MP_4$) and ($MP_5$), which can be remotely updated through the communication subsystem. Each of these modules has different functions or features depending on the bioimpedance measurement application. A pre-configured protocol defines the processing modules that can be activated, the activation sequence and the operating parameters for autonomous operation of the smart sensor. This protocol can be wirelessly re-configured via commands.

a) ($MP_1$) processing module for the estimation of bioimpedance values by a pseudo-demodulation of signals in quadrature.

The result of this processing module is a complex bioimpedance value (modulus and phase) for all operating frequencies. The number and values of frequencies can be set remotely. This module is a novel approach, as a result of the processing method that is needed to derive the values of the bioimpedance, from the variation of the generic quadrature detection system. Moreover, due to the fact that the scheme proposed uses a single multiplier, two frequency sweeps are needed. The ($MP_1$) processing module uses the digital value of ($S_{12}$) from the sensing subsystem. It can be noticed that this signal corresponds to the following equation:

$$S_{12} = A_{12} * \left[ \frac{A_7^2 * A_1^2}{2} - \frac{A_7 * A_1 * A_5 * A_4 * A_B * \cos(\varphi_B - \varphi_6)}{2} \right] \quad [\text{Equation 1}]$$

In this equation, $A_7$, $A_4$ and $A_1$ are known parameters, $A_5$, $A_{12}$ and $\varphi_6$ are configurable parameters and $A_B$ and $\varphi_B$ are the variables that will provide the respective bioimpedance values. This is defined as the ratio between the voltage and the current produced, in such a way that in the case of the bioimpedance smart sensor, the impedance phase corresponds to $\varphi_B$ and the modulus can be expressed as:

$$|Z| = \frac{A_B}{A_I} \quad [\text{Equation 2}]$$

Where $A_I$ is also a parameter of known value.
The execution of the processing module to estimate bioimpedance values is divided into the following stages:

- Stage I.a - First pseudo-demodulation in phase: in this stage the gain values $A_5$ and $A_{12}$ are configured to take unit value. The module ($M_8$) is set in position A. In addition, the ($M_6$) module of generation of internal signal is configured for the phase $\varphi_6$ taking zero value. In this case, the ($S_{12}$) signal responds to the following expression:

$$S_{12}|_{I.a} = \frac{A_7^2 * A_1^2}{2} - \frac{A_7 * A_1 * A_4 * A_B * \cos(\varphi_B)}{2} \quad [\text{Equation 3}]$$

A frequency sweep is performed, setting in each frequency the modules ($M_1$) and ($M_6$) of signal generation and internal signal with their corresponding frequencies. At each frequency the average value of the sum of ($N_a$) measurements of ($S_{12}|_{I.a}$) is stored, where ($N_a$) can also take unit value. In addition, for each frequency, the first measurement is performed after a waiting time ($T_1$) so that the signal ($S_{12}|_{I.a}$) reaches its stationary value. Between measurements there is a waiting time ($T_2$).
- Stage II.a - First pseudo-demodulation in quadrature: in this stage the gains $A_5$ and $A_{12}$ hold their unit value. The module ($M_8$) remains set to the A position. In addition, the module of internal signal generation ($M_6$) is configured so that the phase $\varphi_6$ takes the value $\pi/2$. In this case, the ($S_{12}$) signal responds to the following expression:

$$S_{12}|_{II.a} = \frac{A_7{}^2 * A_1{}^2}{2} - \frac{A_7 * A_1 * A_4 * A_B * \sin(\varphi_B)}{2} \qquad \text{[Equation 4]}$$

A frequency sweep is performed, setting in each frequency the modules $(M_1)$ and $(M_6)$ of signal generation and internal signal with their corresponding frequencies. At each frequency, the average value of the sum of $(N_a)$ measures of $(S_{12}|_{II.a})$ is stored, where $(N_a)$ can also take unit value. In addition, for each frequency, the first measurement is performed after a waiting time $(T_1)$ so that the signal $(S_{12}|_{II.a})$ reaches its stationary value. Between measurements there is a delay $(T_2)$.

- Stage III.a - First approximation to the bioimpedance values: in this stage, from the values stored in stages I.a and II.a, the magnitude and phase of bioimpedance at each frequency is calculated. To do this, firstly the variables $(C_1)$ and $(C_2)$ are calculated. They are defined as:

$$C_1 = \frac{2 * \left[\dfrac{A_7{}^2 * A_1{}^2}{2} - S_{12}|_{I.a}\right]}{A_7 * A_1 * A_4} \qquad \text{[Equation 5]}$$

$$C_2 = \frac{2 * \left[\dfrac{A_7{}^2 * A_1{}^2}{2} - S_{12}|_{II.a}\right]}{A_7 * A_1 * A_4} \qquad \text{[Equation 6]}$$

It can be shown that from the expressions of $(S_{12}|_{I.a})$ and $(S_{12}|_{I.a})$, described in stages I.a and II.a, the module of bioimpedance can be expressed as:

$$|Z| = \frac{A_4 * A_B}{A_I} \qquad \text{[Equation 7]}$$

In which $(A_B)$ is equal to:

$$A_B = \sqrt{C_1{}^2 + C_2{}^2} \qquad \text{[Equation 8]}$$

And the bioimpedance phase as:

$$\varphi_B = \tan^{-1}\left(\frac{C_2}{C_1}\right) \qquad \text{[Equation 9]}$$

Such equations are used to find a first approach to bioimpedance values.

1. Processing module to increase the accuracy of bioimpedance values by a second pseudo-demodulation of signal in quadrature (MP2).

Another novel feature of the processing subsystem derives from using a second schema of signal demodulation in quadrature complementary to that performed in $(MP_1)$. The used schema is a novel method allowing increasing the dynamic range of signal to noise, by increasing the precision degree in the digital signal provided by the module $(M_{13})$ by adjusting two gain stages. The execution of this processing module may not be necessary for all or some frequencies if the sampling error related to the analog-to-digital conversion is kept below 1% of the detected signal. The processing module $(MP_2)$ is divided into the following stages:

- Stage I.b - Second pseudo-demodulation in phase: in this stage, a process equivalent to that carried out in stage I.a of processing module $(MP_1)$ is performed, but adapting the values of the gains $A_5$ and $A_{12}$ in accordance with a criterion that maximizes the signal to noise ratio of $(S_{12})$ in a context of analog-to-digital conversion. Such optimization criterion is defined below:

Procedure for optimizing the accuracy of the processing module (MP$_2$):

The objective of this procedure is to increase the measurement accuracy by reducing the sampling error caused by the analog-to-digital conversion. According to [Equation 1], the signal (S$_{12}$) can be defined as follows:

$$S_{12} = A_{12} * [C_3 - C_4] \qquad \text{[Equation 10]}$$

Where (C$_3$) is defined as:

$$C_3 = \frac{A_7{}^2 * A_1{}^2}{2} \qquad \text{[Equation 11]}$$

and (C$_4$) as:

$$C_4 = \frac{A_7 * A_1 * A_5 * A_4 * A_B * \cos(\varphi_B - \varphi_6)}{2} \qquad \text{[Equation 12]}$$

Regarding the proposed criterion for accuracy optimization, (A$_7$) must be pre-configured in such a way that the parameter (C$_3$) is close to the maximum allowable value of the module of analog-to-digital conversion (M$_{13}$). The next step is to define a value for the gain (A$_5$) that approximates the value of the parameter (C$_4$) to the parameter (C$_3$) without exceeding it, thus the difference between (C$_3$) and (C$_4$) will have a small and positive value. The degree of approximation should be such that even with fluctuations of the parameter (C$_4$) the difference between signals is always positive. The last step is to define a value for the gain (A$_{12}$) so that the signal (S$_{12}$) approaches the maximum allowable value of the module of analog-to-digital conversion (M$_{13}$). The degree of approximation should be such that even with fluctuations of the signal (S$_{12}$), it always remains below the maximum allowable value of the module of analog-to-digital conversion (M$_{13}$).

- Stage II.b - Second pseudo-demodulation in quadrature: in this stage a process equivalent to that carried out in stage II.a is performed, but adapting the values of the gains A$_5$ and A$_{12}$ in accordance with the optimization criterion described on stage I.b that maximizes the signal to noise ratio of (S$_{12}$) in a context of analog-to-digital conversion.

- Stage III.b - Second approximation to the bioimpedance values: in this stage a process equivalent to that carried out in stage III.b takes place but from the values stored in stages I.b and II.b. To do this, first the variables (C$_5$) and (C$_6$) are calculated. They are defined as

$$C_5 = \frac{A_7{}^2 * A_1{}^2 - \dfrac{2 * S_{12}|_{I.b}}{A_{12}}}{A_7 * A_1 * A_5 * A_4} \qquad \text{[Equation 13]}$$

$$C_6 = \frac{A_7{}^2 * A_1{}^2 - \dfrac{2 * S_{12}|_{II.b}}{A_{12}}}{A_7 * A_1 * A_5 * A_4} \qquad \text{[Equation 14]}$$

The modulus of bioimpedance can be expressed as:

$$|Z| = \frac{A_B}{A_I} \qquad \text{[Equation 15]}$$

In which (A$_B$) is equal to:

$$A_B = \sqrt{C_5{}^2 + C_6{}^2} \qquad \text{[Equation 16]}$$

And the bioimpedance phase as:

$$\varphi_B = \tan^{-1}\left(\frac{C_6}{C_5}\right) \qquad \text{[Equation 17]}$$

Such equations are used in this second approximation of higher accuracy to the bioimpedance values.

2. Processing module for the estimation of bioimpedance values by successive approximations ($MP_3$).

Another novel feature of the processing subsystem derives from using a third processing schema for estimating the values of bioimpedance, which can be used in isolation or complementary to the previous two modules. The schema used in this case is a novel method based on successive approximations that estimates bioimpedance with a great accuracy. Furthermore, as the technique differs from that used in the two previous modules, it allows a second validation of the obtained values.

The proposed method is based on a sequence of successive approximations to the module and phase of bioimpedance, setting as right values those that minimize a certain error function. Such error function weights the mismatch between the detected signal and that which would be detected if the bioimpedance took the analyzed values. This error function reaches a minimum when the configuration parameters of the smart sensor generate an internal signal equivalent in module and phase to the signal at the output of the module ($M_5$). This situation results in a null value in the signal ($S_{12}$) defined in [Equation 1], which is the only available value of the sensing subsystem (in this schema the module ($M_8$) is also configured to position A). The approximation procedure to the bioimpedance values will consist in modifying the configuration parameters of the smart sensor until the following condition is met:

$$S_{12} = A_{12} * [C_3 - C_4] = 0 \qquad \text{[Equation 18]}$$

In which ($C_3$) and ($C_4$) take the values previously defined:

$$C_3 = \frac{A_7{}^2 * A_1{}^2}{2} \qquad \text{[Equation 19]}$$

$$C_4 = \frac{A_7 * A_1 * A_5 * A_4 * A_B * \cos(\varphi_B - \varphi_6)}{2} \qquad \text{[Equation 20]}$$

However, the process must be done to ensure that the phase equality is met firstly, since for each value of the difference ($\varphi_B$-$\varphi_6$) there is a value of the parameter ($A_5$) for which the equality is satisfied.

Regarding the proposed procedure, from an initial value of the gain ($A_5$) the algorithm searches in each frequency through successive approximations the phase $\varphi_6$ that minimizes the value of ($S_{12}$). If necessary, the system will enter in an iterative process of increasing the gain ($A_5$) and searching the phase that minimizes the value of ($S_{12}$). During this process, a progressive increase of the gain ($A_{12}$) can be made in order to increase the accuracy of the estimation of module and phase. Any variation of the parameters must be made so that the value of ($S_{12}$) remains positive and below the maximum allowable value of the module of analog-to-digital conversion ($M_{13}$). The algorithm will decide whether the right value has been reached when the value of ($S_{12}$) is below a certain threshold weighted by the value of the gain ($A_5$). In this case, the modulus of the bioimpedance can be approached as:

$$|Z| = \frac{A_B}{A_I} \qquad \text{[Equation 21]}$$

In which ($A_B$) is equal to:

$$A_B = \frac{A_7 * A_1}{A_5 * A_4} \qquad \text{[Equation 22]}$$

And the phase of the bioimpedance as:

$$\varphi_B = \varphi_6 \qquad \text{[Equation 23]}$$

This method of estimating the modulus and phase of the bioimpedance can be complementary to that performed in previous stages. In that case, it will start from initial values for ($A_5$) and ($\varphi_6$) close to the values derived from the modulus and phase obtained in previous stages. It can also be performed in isolation, by using when it is possible as input parameters the initial values for ($A_5$) and ($\varphi_6$) close to those obtained for the previous frequency, since the bioimpedance measurements in nearby frequencies take similar values. Thus the number of global iterations in the process is reduced.

Finally, the values for the modulus and phase of the bioimpedance in each multi-frequency measurement are proposed as a function of the estimation provided by one or several ($MP_1$), ($MP_2$) or ($MP_3$) processing modules.

3. Processing module for the monitoring of the modulus and phase of the impedance at a single frequency ($MP_4$)

Another novel feature of the processing subsystem derives from using a fourth processing schema allowing tracking of the modulus and phase of the bioimpedance at a particular frequency. This module can be executed when no measurement of multi-frequency bioimpedance is being performed. A sampling rate will define the time between consecutive data estimations. Each estimate will be stored in the data storage subsystem. Such data can be sent wirelessly in real time through the communication system. It is also possible a deferred submission when the size of stored data is high enough, when it is required by a command, or when the timing subsystem generates a send event. To estimate the modulus and phase of the bioimpedance any of the previous methods ($MP_1$), ($MP_2$) or ($MP_3$) can be used, in isolation or complementary, but in a special setting that analyzes exclusively the operating frequency. Furthermore, starting data used by the processing modules ($MP_2$) and ($MP_3$) may be set to take as a reference the values of magnitude and phase of bioimpedance from the previous sample. The sampling frequency will depend on the particular application of the bioimpedance sensor and will be a configurable parameter by command. It is also possible that an algorithm recursively searches the frequency associated with an increased sensitivity to the phenomenon causing variations in the bioimpedance. The processing module ($MP_4$) can be activated locally on the smart sensor using a push button, remotely by sending a command, or self-activated in time instants pre-configured by commands.

4. Processing module for the monitoring of the impedance modulus at a single frequency ($MP_5$).

Another novel feature of the processing subsystem derives from using a fifth processing schema allowing tracking the magnitude of the bioimpedance at a particular frequency. The functionality of this processing module is similar to the processing module ($MP_4$) although it is not equivalent since it provides no phase of the bioimpedance. In this sense, processing modules ($MP_4$) and ($MP_5$) will not be active in one device at the same time and, regarding the particular application of bioimpedance measurement, it is possible that neither of them are activated. The main difference between these two modules is the schema used for the sensing subsystem. In the processing module ($MP_4$), the module ($M_8$) is set on position A. However, in the processing module ($MP_5$), the module ($M_8$) is set on position B. This feature allows reducing the sampling time needed to taking a bioimpedance measurement, which can be useful in those cases where only the tracking of bioimpedance module is necessary. To perform such estimations the gain ($A_5$) from the module ($M_5$) is set to 0. In this case, the value of the modulus of the bioimpedance responds to the following equation:

$$|Z| = \sqrt{\frac{2 * S_{12}}{A_{12} * A_4{}^2 * A_I{}^2}} \qquad \text{[Equation 24]}$$

This module can be executed when no measure of multifrequency bioimpedance is being performed. A sampling rate will define the time between consecutive data estimations. Each estimate will be stored in the data storage subsystem. Such data can be sent wirelessly in real time through the communication system. It is also possible a deferred submission when the size of stored data is high enough, when it is required by a command, or when the timing subsystem generates a send event. The operating frequency for tracking will depend on the particular application of the bioimpedance sensor. It is also possible that an algorithm recursively searches the frequency associated with an increased sensitivity to the phenomenon causing variations in the bioimpedance. The processing module ($MP_5$) can be activated locally on the smart sensor using a push button, remotely by sending a command, or self-activated in time instants pre-configured by commands.

**Description of the drawings**

To complement the description that is being made and to ease the understanding of the features of the invention, supporting a preferred embodiment of its practical realization, a set of drawings accompanies as an integral part of the description, in which with illustrative and non-limitative character, the following has been represented:

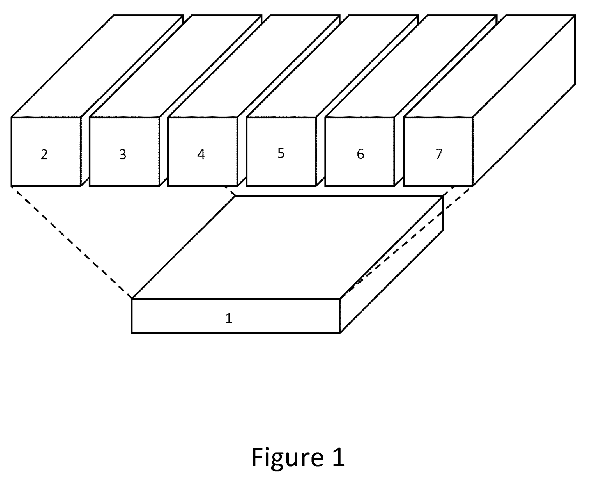

Figure 1.- Basic architecture of "Bioimpedance Smart Sensor for Biomedical Applications"

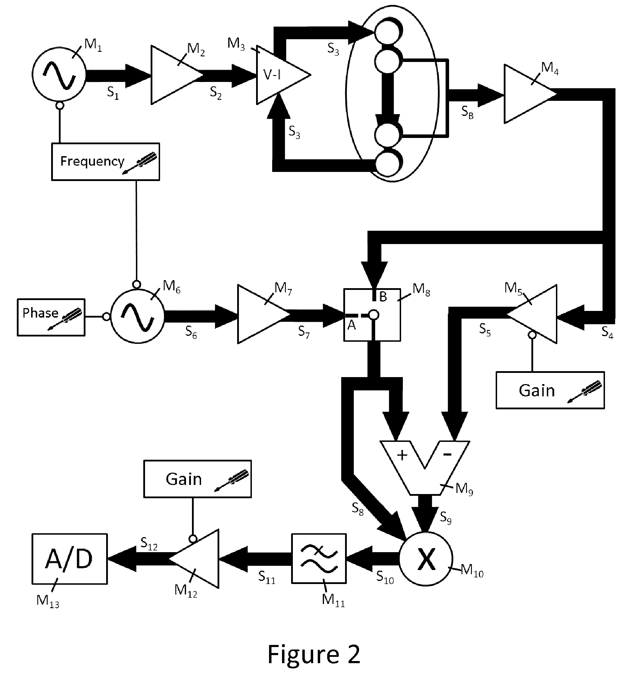

Figure 2.- Sensing subsystem.

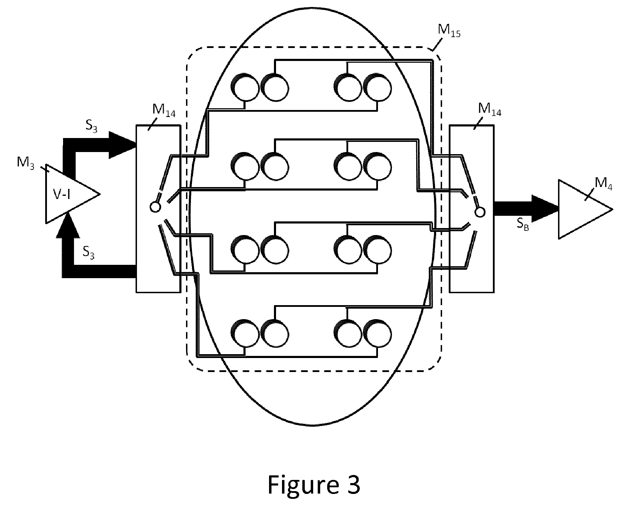

Figure 3.- Detail in sensing subsystem of the module of multiplexing ($M_{14}$) and module of coupling ($M_{15}$).

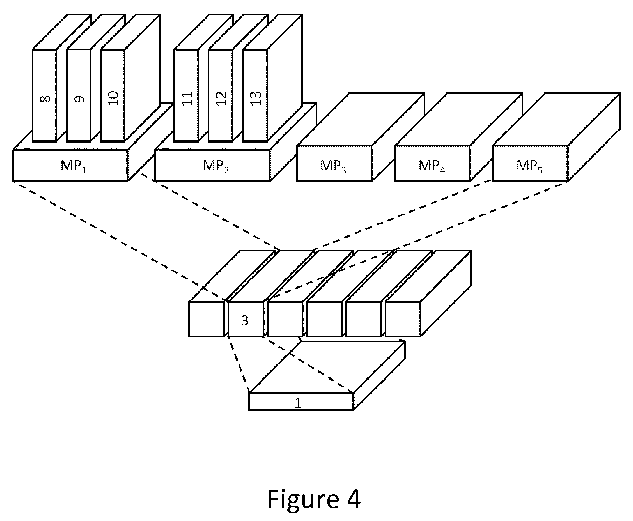

Figure 4.- Processing subsystem.

In the above figures the following constituent elements can be highlighted:

1.    System that brings together all the constituent subsystems.
2.    Sensing subsystem.
3.    Processing subsystem.
4.    Communication subsystem.
5.    Data storage subsystem.
6.    Timing subsystem.
7.    Power subsystem.
8.    Stage I.a - First pseudo-demodulation in phase.
9.    Stage II.a - First pseudo-demodulation in quadrature.
10.   Stage III.a - First approximation to the bioimpedance values.
11.   Stage I.b - Second pseudo-demodulation in phase.
12.   Stage II.b - Second pseudo-demodulation in quadrature.
13.   Stage III.b - Second approximation to the bioimpedance values.

**EXAMPLE OF PREFERRED EMBODIMENT**

In a preferred embodiment of the "Bioimpedance Smart Sensor for Biomedical Applications", the device is used to perform a bioimpedance characterization of a body section (arm, trunk or limb), which can be performed with all the subsystems, except the module of coupling, integrated within a single housing. Two 9V batteries are fundamental part of the energy subsystem. As part of the same subsystem, two regulators generate 5V and -5V stable voltages to power the analog part of the sensing subsystem. Another 3V regulator is responsible for stabilizing the operating voltage of the digital components. The processing subsystem is integrated in a microprocessor with an arithmetic logic unit of 8 bits operating at 4 MHz. The different processing modules are programmed into the flash memory of the microprocessor, which has a capacity of 16 Mbytes. The communications subsystem is supported by a transceiver that meets the specifications of the IEEE 802.15.4 standard. Another model of the device uses a transceiver based on the IEEE 802.15.1 standard. To develop the data storage subsystem, the SRAM memory (768 bytes) and the EEPROM memory (256 bytes) of the microprocessor are used. The timing subsystem is also implemented in the microprocessor program code. An external 32.768 kHz crystal and one of the timers of the microprocessor are used to manage the timing in real time. The module ($M_1$) is a programmable oscillator which uses the technique of Direct Digital Synthesis (DDS) of frequencies. The DDS can generate any frequency between 0.19 Hz and 25 MHz with a resolution of 0.19 Hz and a stability of 40 ppm. The frequency and phase of the generated signal are controlled through a serial data interface. The abrupt changes in the signal due to the digital sampling are smoothed through a low pass filter with a cuttoff frequency high enough so as not to affect the generated signals. Analog schemes based on operational amplifiers are used for the amplification modules and the voltage-current conversion module.

It is established as design specification a maximum error of 1% of the estimated value of the complex bioimpedance measurement. In this sense, all components employed (operational amplifiers, resistors, or other) have characteristics such as to ensure a maximum error below 1% in the range of device operation. For the implementation of the ($M_4$) module an instrumentation amplifier is used, which is based on operational amplifiers with a common-mode rejection ratio of 47 dB and an input impedance of 1 MΩ. Gains of the variable gain amplifiers are configured through digital potentiometers. To implement the module ($M_{12}$) other DDS is used with the same characteristics as that used for the module of injection signal generation. As any difference in the frequencies generated by both modules, no matter how small, produces a continuous drift of the offset between the two signals, only one 50 MHz crystal is used as time reference for both DDS with a stability of 20 ppm. Thus, both devices have exactly the same frequency and the offset programmed between the signals of the two modules remain constant over time. For the subtraction module ($M_8$) a scheme based on operational amplifiers is also employed and for the module ($M_9$) a four-quadrant multiplier integrated circuit is used. Module ($M_{10}$) is an active low-pass filter of second order based on operational amplifiers with a cutoff frequency of 13.8 Hz. To implement the module ($M_{12}$) one of the 10-bit Analog-to-Digital converters of the microcontroller is used, whose maximum voltage is configured to provide a resolution of 1.17 mV. Module ($M_{13}$) uses several digitally controlled analog switches. These switches allow performing two simultaneous measurements of bioimpedance in two different sections of the biological medium to be measured (two channels). Two connectors in the housing enable the connection of the measurement cables, one per channel. Each cable has a connector at one end, which is coupled to the connector of the housing. At the other end the cable is divided into four different lines, which terminates in metal clips for its connection to the electrodes. Cables and lines have a metal mesh to protect against interference signals. The lines are electrically isolated from the protective mesh, and the mesh is in turn electrically insulated from the outside. The cables are flexible and each one has a length of 1.5 meters. Although the device can be adapted to different types of electrodes, depending on the application, circular ECG clip electrodes are preferably used for bioimpedance measurements on the human body.

It is not considered necessary to extend this description so that any expert in the field understands the scope of the invention and the advantages derived from it. The devices that compose it, the technical solutions adopted or even its application may be susceptible to variation as long as this does not alter the essence of the invention.

## Claims

1.  Bioimpedance Smart Sensor for Biomedical Applications (1) **characterized by** being performed from the following subsystems:

    a) A sensing subsystem (2) that incorporates the necessary hardware to perform bioimpedance measurements.
    b) A processing subsystem (3) that integrates the hardware, software and firmware elements responsible for the proper activation and configuration of the sensing subsystem modules and the processing associated with the estimation of the bioimpedance values.
    c) A communication subsystem (4) that integrates the hardware, software and firmware elements responsible for the bidirectional wireless communications of the device.
    d) A data storage subsystem (5) responsible for the proper storage of data.
    e) A timing subsystem (6) responsible for the maintenance of a timing system in real-time, the allocation of the temporal instant to perform each measurement and the notification to the processing subsystem of the correct instant for the realization of the operations.
    f) A power subsystem (7) that is responsible for providing the supply voltages necessary for the proper operation of all the subsystems.

2.  Bioimpedance Smart Sensor for Biomedical Applications (1) according to claim 1, with the sensing subsystem **characterized by** comprising:

    a) module ($M_1$) that generates a sinusoidal voltage signal ($S_1$) with fixed amplitude ($A_1$) and configurable frequency ($f_1$).
    b) module ($M_2$) that amplifies signal ($S_1$) with gain ($A_2$) to generate signal ($S_2$).
    c) module ($M_3$) that converts voltage signal ($S_2$) to current signal ($S_3$) with fixed current amplitude ($A_I$) and the same frequency ($f_1$), which is injected into the biological medium through two electrodes (distal electrodes).
    d) module ($M_4$) that amplifies the voltage detected by the other two electrodes (proximal electrodes) with gain ($A_4$), generating signal ($S_4$).
    e) module ($M_5$) that amplifies signal ($S_4$) with configurable gain ($A_5$) to generate signal ($S_5$).
    f) module ($M_6$) that generates a sinusoidal voltage signal ($S_6$) with the same amplitude value ($A_1$) and the same frequency ($f_1$) than signal ($S_1$), but with a configurable phase difference ($\varphi_6$) between them.
    g) module ($M_7$) that amplifies signal ($S_6$) with gain ($A_7$) to generate signal ($S_7$).

h) multiplexer module ($M_8$) whose output signal ($S_8$) can be configured to correspond to signal ($S_7$) in position A or signal ($S_4$) in position B.

i) module of subtraction ($M_9$) that generates signal ($S_9$) as a result of subtraction signal ($S_5$) from signal ($S_8$).

j) module ($M_{10}$) that generates signal ($S_{10}$) as a result of the multiplication of signal ($S_8$) and signal (S9).

k) module ($M_{11}$) that generates signal ($S_{11}$) as a result of low-pass filtering signal ($S_{10}$).

l) module ($M_{12}$) that amplifies signal ($S_{11}$) with a configurable gain ($A_{12}$) to generate signal ($S_{12}$).

m) module ($M_{13}$) responsible for the conversion of the analog signal ($S_{12}$) into digital signals.

n) module ($M_{14}$) allowing multiplexing the injected current and the voltage detected to estimate the bioimpedance of different body sections.

o) module ($M_{15}$) that comprises the sensing system electrodes, for both current injection (distal electrodes) and voltage detection (proximal electrodes), and the cables and connectors that act as links between these electrodes and module ($M_{14}$).

3. Bioimpedance Smart Sensor for Biomedical Applications (1) according to claim 1, with the processing subsystem **characterized by** comprising:

a) processing module for the estimation of bioimpedance values by a pseudo-demodulation of signals in quadrature ($MP_1$).

b) processing module to increase the accuracy of bioimpedance values by a second pseudo-demodulation of signal in quadrature ($MP_2$).

c) processing module for the estimation of bioimpedance values by successive approximations ($MP_3$), establishing as correct values those that minimize the mismatch between the signal detected and the signal that would be detected if the bioimpedance had the analyzed values.

d) processing module for the monitoring of the modulus and phase of the impedance at a single frequency ($MP_4$), wherein a sampling rate defines the time between consecutive estimations, using any of the above modules ($MP_1$), ($MP_2$) or ($MP_3$), alone or in a complementary manner, in a special configuration that only analyzes the operating frequency.

e) processing module for the monitoring of the impedance modulus at a single frequency ($MP_5$), wherein a sampling rate defines the time between consecutive estimations.

4. Bioimpedance Smart Sensor for Biomedical Applications (1) according to claim 1 and 3, with processing module ($MP_5$) **characterized by** using the equation shown below to calculate the bioimpedance modulus, with module ($M_8$) configured in position B and gain ($A_5$) set to 0,

$$|Z| = \sqrt{\frac{2 * S_{12}}{A_{12} * A_4{}^2 * A_I{}^2}} \qquad \text{[Equation 37]}$$

5. Bioimpedance Smart Sensor for Biomedical Applications (1) according to claim 1, 3 and 4 **characterized in that** its bioimpedance measurements can be activated locally in the smart sensor using a push button, remotely by sending a command, or they may be self-activated in a series of time instants pre-configured through commands.

6. Bioimpedance Smart Sensor for Biomedical Applications (1) according to claim 1, provided with a processing subsystem according to claims 3, 4 and 5, **characterized in that** processing modules ($MP_1$), ($MP_2$), ($MP_3$), ($MP_4$) and ($MP_5$) can be remotely updated through the communications subsystem.

7. Bioimpedance Smart Sensor for Biomedical Applications (1) according to claim 1, provided with a processing subsystem according to claims 3, 4, 5 and 6, **characterized by** the autonomous operation of the smart sensor according to a protocol, re-configurable by commands, which defines the processing modules that can be activated, the activation sequence and their operating parameters.

8. Method used in ($MP_1$) processing module, comprised within the processing subsystem of the Bioimpedance Smart Sensor for Biomedical Applications (1), **characterized by** comprising the following operations:

a) A first pseudo-demodulation in phase (Stage I.a) (8) in which the value of signal ($S_{12}$) is stored for each frequency ($S_{12}|_{I.a}$), with the sensing subsystem configured in a way in which $A_5$ and $A_{12}$ take the value one, $\varphi_6$ takes the value 0 and ($M_8$) is in position A.

b) A first pseudo-demodulation in quadrature (Stage II.a) (9) in which the value of signal ($S_{12}$) is stored for each frequency ($S_{12}|_{II.a}$), with the sensing subsystem configured in such a way that $A_5$ and $A_{12}$ take unit value, $\varphi_6$ takes the value $\pi/2$ and ($M_8$) is in position A.

c) A first approximation to the bioimpedance values (Stage III.a) (10) in which the modulus and phase of the impedance for each frequency is calculated from the values stored in the I.a y II.a stages using the following equations:

$$Modulus = \frac{A_4 * A_B}{A_I} \qquad \text{[Equation 25]}$$

$$Fase = \tan^{-1}\left(\frac{C_{II}}{C_I}\right) \qquad \text{[Equation 26]}$$

considering that:

$$C_I = \frac{2 * \left[\dfrac{A_7{}^2 * A_1{}^2}{2} - S_{12}|_{I.a}\right]}{A_7 * A_1 * A_4} \qquad \text{[Equation 27]}$$

$$C_{II} = \frac{2 * \left[\dfrac{A_7{}^2 * A_1{}^2}{2} - S_{12}|_{II.a}\right]}{A_7 * A_1 * A_4} \qquad \text{[Equation 28]}$$

$$A_B = \sqrt{C_I{}^2 + C_{II}{}^2} \qquad \text{[Equation 29]}$$

**9.** Method used in (MP$_2$), comprised within the processing subsystem of the Bioimpedance Smart Sensor for Biomedical Applications (1), **characterized by** comprising the following operations:

a) A second pseudo-demodulation in phase (Stage I.b) (11) in which a process equivalent to that performed in claim 8 takes place, but adapting the values of gains $A_5$ and $A_{12}$ according to a criterion that maximizes the signal to noise ratio ($S_{12}$).

b) A second pseudo-demodulation in quadrature (Stage II.b) (12) in which a process equivalent to that performed during stage II.a of claim 8 takes place, but adapting the values of gains $A_5$ and $A_{12}$ according to a criterion that maximizes the signal to noise ratio ($S_{12}$).

c) A second approximation to the bioimpedance values (Stage III.b) (13) in which, from the values stored in I.b and II.b stages, the modulus and phase of the impedance for each frequency is calculated using the following equations:

$$Modulus = \frac{A_B}{A_I} \qquad \text{[Equation 30]}$$

$$Phase = \tan^{-1}\left(\frac{C_{IV}}{C_{III}}\right) \qquad \text{[Equation 31]}$$

considering that:

$$C_{III} = \frac{A_7{}^2 * A_1{}^2 - \dfrac{2 * S_{12}|_{I.b}}{A_{12}}}{A_7 * A_1 * A_5 * A_4} \qquad \text{[Equation 32]}$$

$$C_{IV} = \frac{A_7{}^2 * A_1{}^2 - \dfrac{2 * S_{12}|_{II.b}}{A_{12}}}{A_7 * A_1 * A_5 * A_4} \qquad \text{[Equation 33]}$$

$$A_B = \sqrt{C_{III}{}^2 + C_{IV}{}^2} \qquad \text{[Equation 34]}$$

10. Method to maximize the signal to noise ratio ($S_{12}$) in the processing module ($MP_2$), comprised within the processing subsystem of the Bioimpedance Smart Sensor for Biomedical Applications (1) according to claim 9, **characterized by** executing the following operations:

   a) Setting the gain ($A_7$) so that the parameter (Cv) is close to the maximum allowable value of module ($M_{13}$), considering that:

$$C_V = \frac{A_7{}^2 * A_1{}^2}{2} \qquad \text{[Equation 35]}$$

   b) Defining a value for the gain ($A_5$) that approximates the value of the parameter ($C_{VI}$) to the parameter (Cv) so that the difference (Cv) - ($C_{VI}$) is always positive, taking into account that:

$$C_{VI} = \frac{A_7 * A_1 * A_5 * A_4 * A_B * \cos(\varphi_B - \varphi_6)}{2} \qquad \text{[Equation 36]}$$

   c) Defining a value for the gain ($A_{12}$) so that the signal ($S_{12}$) approximates the maximum permissible value of the Analog-to-Digital conversion module ($M_{13}$).

11. Method to obtain the modulus and phase values for each multi-frequency bioimpedance measurement of the Bioimpedance Smart Sensor for Biomedical Applications (1) according to claims 8, 9 and 10, **characterized in that** they are proposed as a function of the estimations provided by one or more of the processing modules ($MP_1$), ($MP_2$) or ($MP_3$).

Figure 1

Figure 2

Figure 3

Figure 4

# EP 3 066 981 A1

## INTERNATIONAL SEARCH REPORT

| | International application No. |
|---|---|
| | PCT/ES2014/070822 |

### A. CLASSIFICATION OF SUBJECT MATTER

***A61B5/05*** (2006.01)

According to International Patent Classification (IPC) or to both national classification and IPC

### B. FIELDS SEARCHED

Minimum documentation searched (classification system followed by classification symbols)

A61B

Documentation searched other than minimum documentation to the extent that such documents are included in the fields searched

Electronic data base consulted during the international search (name of data base and, where practicable, search terms used)

EPODOC, INVENES

### C. DOCUMENTS CONSIDERED TO BE RELEVANT

| Category* | Citation of document, with indication, where appropriate, of the relevant passages | Relevant to claim No. |
|---|---|---|
| X | US 2010168530 A1 (CHETHAM SCOTT ET AL.) 01/07/2010, paragraph [0007]; paragraphs[0020 - 0699]; | 1, 2, 8 |
| Y | | 9 |
| A | | 3-7, 10-11 |
| X | US 2010312188 A1 (ROBERTSON TIMOTHY ET AL.) 09/12/2010, figure 2, paragraphs[0066 - 0299]; | 1, 2, 8 |
| Y | | 9 |
| X | US 2007166011 A1 (WANG MI WANG MI ET AL.) 19/07/2007, paragraphs[0001 - 0087]; | 1 |
| X | EP 1275342 A2 (CARDIODYNAMICS INTERNAT CORP) 15/01/2003, paragraphs[0013 - 0117]; | 1 |

☐ Further documents are listed in the continuation of Box C.          ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | |
| | "&" document member of the same patent family |

| Date of the actual completion of the international search | Date of mailing of the international search report |
|---|---|
| 25/03/2015 | **(25/03/2015)** |
| Name and mailing address of the ISA/ | Authorized officer |
| | A. Casado Fernández |
| OFICINA ESPAÑOLA DE PATENTES Y MARCAS | |
| Paseo de la Castellana, 75 - 28071 Madrid (España) | |
| Facsimile No.: 91 349 53 04 | Telephone No. 91 3496871 |

Form PCT/ISA/210 (second sheet) (July 2009)

## INTERNATIONAL SEARCH REPORT

Information on patent family members

| International application No. |
| --- |
| PCT/ES2014/070822 |

| Patent document cited in the search report | Publication date | Patent family member(s) | Publication date |
| --- | --- | --- | --- |
| US2010168530 A1 | 01.07.2010 | US2015002177 A1 | 01.01.2015 |
| | | JP2011502014 A | 20.01.2011 |
| | | JP5513396B B2 | 04.06.2014 |
| | | US2011025348 A1 | 03.02.2011 |
| | | US8836345 B2 | 16.09.2014 |
| | | JP2010515492 A | 13.05.2010 |
| | | JP5400618B B2 | 29.01.2014 |
| | | CA2704061 A1 | 14.05.2009 |
| | | JP2010510835 A | 08.04.2010 |
| | | JP5372768B B2 | 18.12.2013 |
| | | AU2008324750 A1 | 14.05.2009 |
| | | AU2008324750B B2 | 16.01.2014 |
| | | US2010100003 A1 | 22.04.2010 |
| | | US8594781 B2 | 26.11.2013 |
| | | CA2675438 A1 | 24.07.2008 |
| | | CA2670293 A1 | 05.06.2008 |
| | | AU2008207332 A1 | 24.07.2008 |
| | | AU2008207332B B2 | 23.05.2013 |
| | | AU2007327573 A1 | 05.06.2008 |
| | | AU2007327573B B2 | 18.07.2013 |
| | | EP2211714 A1 | 04.08.2010 |
| | | WO2009059351 A1 | 14.05.2009 |
| | | EP2106241 A1 | 07.10.2009 |
| | | EP2106241 A4 | 18.07.2012 |
| | | EP2091425 A1 | 26.08.2009 |
| | | EP2091425 A4 | 25.07.2012 |
| | | WO2008064426 A1 | 05.06.2008 |
| | | WO2008086565 A1 | 24.07.2008 |
| US2010312188 A1 | 09.12.2010 | JP2014208301 A | 06.11.2014 |
| | | US2014236077 A1 | 21.08.2014 |
| | | HK1162903 A1 | 27.06.2014 |
| | | US2014051946 A1 | 20.02.2014 |
| | | US2014009262 A1 | 09.01.2014 |
| | | TW201320961 A | 01.06.2013 |
| | | JP2013010029 A | 17.01.2013 |
| | | JP5591298B B2 | 17.09.2014 |
| | | CN102885615 A | 23.01.2013 |
| | | CA2792224 A1 | 01.07.2010 |
| | | JP2012511969 A | 31.05.2012 |
| | | JP5143290B B2 | 13.02.2013 |
| | | US2012101430 A1 | 26.04.2012 |
| | | US8545436 B2 | 01.10.2013 |
| | | SG172165 A1 | 28.07.2011 |
| | | CN102316795 A | 11.01.2012 |
| | | CN102316795B B | 30.10.2013 |
| | | AU2009330321 A1 | 14.07.2011 |
| | | AU2009330321B B2 | 07.03.2013 |
| | | KR20110094147 A | 19.08.2011 |
| | | KR101146555B B1 | 25.05.2012 |

Form PCT/ISA/210 (patent family annex) (July 2009)

## INTERNATIONAL SEARCH REPORT

Information on patent family members

| International application No. |
|---|
| PCT/ES2014/070822 |

| Patent document cited in the search report | Publication date | Patent family member(s) | Publication date |
|---|---|---|---|
| | | TW201034625 A | 01.10.2010 |
| | | TWI424832B B | 01.02.2014 |
| | | IL213491 A | 28.11.2013 |
| | | CA2747156 A1 | 01.07.2010 |
| | | CA2747156 C | 22.10.2013 |
| | | US8114021 B2 | 14.02.2012 |
| | | WO2010075115 A2 | 01.07.2010 |
| | | WO2010075115 A3 | 23.09.2010 |
| | | EP2358271 A2 | 24.08.2011 |
| | | EP2358271 A4 | 18.12.2013 |
| US2007166011 A1 | 19.07.2007 | US7983853 B2 | 19.07.2011 |
| | | WO2005022138 A1 | 10.03.2005 |
| | | GB2422676 A | 02.08.2006 |
| | | GB2422676 B | 16.07.2008 |
| EP1275342 A2 | 15.01.2003 | US6602201 B1 | 05.08.2003 |
| | | US7251524 B1 | 31.07.2007 |
| | | US7149576 B1 | 12.12.2006 |

Form PCT/ISA/210 (patent family annex) (July 2009)

# EP 3 066 981 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7917202 B
- US 6615077 B
- US 7945317 B
- US 20110275922 A
- US 20060122540 A
- US 20130046165 A
- US 20060004300 A
- US 7457660 B
- US 20050012414 A
- US 20040171963 A
- US 7974691 B
- US 6076015 A
- US 6370424 B
- US 20100081960 A
- EP 2567657 A1
- US 20070142733 A
- US 7706872 B
- US 7930021 B
- US 20050101875 A
- US 6532384 B
- US 7783344 B
- US 5876353 A
- US 6790178 B
- US 20120035432 A
- US 7133716 B, M. Kraemer and P. Chamney
- US 20080086058 A, P. Chamney and P.Wabel
- US 20070027402 A, N. Levin and F. Zhu
- US 6151523 A, J. Rosell, P. Riu, R. Pallas, J. Elvira, and R. Bragos
- US 4807638 A, B. Sramek
- US 5103828 A, B. Sramek
- US 7261697 B, D. Berstein
- US 4836214 A, B. Sramek
- US 5505209 A, W. Reining
- US 5782774 A, A. Shmulewitz
- US 6044294 A, S. Mortazavi, E. Park, G. Bornzin, J. Florio, J. Sholder, and R. Weyant
- US 6161038 A, S. Schookin, V. Zubenko, K. Beliaev, A. Morozov, and W. Yong
- US 4870578 A, V. Vysin and B. Sramek
- US 6022322 A, D. Prutchi
- US 5792668 A, M. Fuller, D. Deamer, M. Iverson, and A. Koshy
- US 6998247 B, S. Monfre, K. Hazen, T. Ruchti, T. Blank, and J. Henderson
- EP 2567657 A, O. Chételat

### Non-patent literature cited in the description

- **L. M. ROA ; D. NARANJO ; L. J. REINA ; A. LARA ; J. A. MILAN ; M. A. ESTUDILLO ; J. S. OLIVA.** Applications of bioimpedance to end stage renal disease (esrd). *Studies in Computational Intelligence,* 2013, vol. 404, 689-769
- **G. ENINIA ; P. ONDZULS.** A portable rheograph for clinical studies. *Biull. Eksp. Biol. Med.,* 1961, vol. 52, 105-107
- **W. KUBICEK ; J. KARNEGIS ; R. PATTERSON ; D. WITSOE ; R. MATTSON.** Development and evaluation of an impedance cardiac output system. *Aerospace medicine,* 1966, vol. 37 (12), 1208-1212
- **R. HENDERSON ; J. WEBSTER.** An impedance camera for spatially specific measurements of the thorax. *IEEE Transactions on Biomedical Engineering,* 1978, vol. 25 (3), 250-254
- **A. LISTON ; R. BAYFORD ; D. HOLDER.** The effect of layers in imaging brain function using electrical impedance tomograghy. *Physiological Measurement,* 2004, vol. 25 (1), 143-158
- **N. LI ; H. XU ; Z. ZHOU ; J. XIN ; Z. SUN ; X. XU.** Reconfigurable bioimpedance emulation system for electrical impedance tomography system validation. *IEEE Transactions on Biomedical Circuits and Systems,* 2013, vol. 7 (4), 460-468
- **J. EDD ; B. RUBINSKY.** Assessment of the viability of transplant organs with 3d electrical impedance tomography. *Annual International Conference of the IEEE Engineering in Medicine and Biology,* 2005
- **P. ABERG ; I. NICANCER ; S. OLLMAR.** Minimally invasive electrical impedance spectroscopy of skin exemplified by skin cancer assessments. *Annual International Conference of the IEEE Engineering in Medicine and Biology,* 2003
- **M. FIEDLER ; L.-C. GERHARDT ; S. DERLER ; G. BISCHOFBERGER ; C. HRNY ; T. MUNZER.** Assessment of biophysical skin properties at different body sites in hospitalized old patients: Results of a pilot study. *Gerontology,* 2012, vol. 58 (6), 513-517

- **C. AMARAL ; B. WOLF.** Effects of glucose in blood and skin impedance spectroscopy. *IEEE AFRICON Conference,* 2007
- **A. TURA ; A. MARAN ; G. PACINI.** Non-invasive glucose monitoring: Assessment of technologies and devices according to quantitative criteria. *Diabetes Research and Clinical Practice,* 2007, vol. 77 (1), 16-40
- **R. LUMBROSO ; N. NAAS ; L. BEITEL ; M. LAWRENCE ; M. TRIFIRO.** Novel bioimpedance sensor for glucose recognition. *Conference Proceedings of the International Symposium on Signals, Systems and Electronics,* 2007
- **N. PIACENTINI ; D. DEMARCHI ; P. CIVERA ; M. KNAFLITZ.** Blood cell counting by means of impedance measurements in a microsystem device. *Proceedings of the 30th Annual International Conference of the IEEE Engineering in Medicine and Biology Society,* 2008
- **G. POP ; L. BISSCHOPS ; B. ILIEV ; P. STRUIJK ; J. HOEVEN ; C. HOEDEMAEKERS.** On-line blood viscosity monitoring in vivo with a central venous catheter, using electrical impedance technique. *Biosensors and Bioelectronics,* 2013, vol. 41 (1), 595-601
- **S. ZHENG ; M. NANDRA ; Y.-C. TAI.** Human blood cell sensing with platinum black electroplated impedance sensor. *Proceedings of the 2nd IEEE International Conference on Nano/Micro Engineered and Molecular Systems,* 2007
- **D. TREBBELS ; D. HRADETZKY ; R. ZENGERLE.** Capacitive on-line hematocrit sensor design based on impedance spectroscopy for use in hemodialysis machines. *Annual International Conference of the IEEE Engineering in Medicine and Biology Society. IEEE Engineering in Medicine and Biology Society,* 2009, vol. 2009, 1208-1211
- **D. DZIONG ; P. BAGNANINCHI ; R. KEARNEY ; M. TABRIZIAN.** Nondestructive online in vitro monitoring of pre-osteoblast cell proliferation within microporous polymer scaffolds. *IEEE Transactions on Nanobioscience,* 2007, vol. 6 (3), 249-258
- **V. SRINIVASARAGHAVAN ; J. STROBL ; M. AGAH.** Bioimpedance rise in response to histone deacetylase inhibitor is a marker of mammary cancer cells within a mixed culture of normal breast cells. *Lab on a Chip - Miniaturisation for Chemistry and Biology,* 2012, vol. 12 (24), 5168-5179
- **I. LAEGREID ; A. BYE ; K. AASAROD ; M. JORDHOY.** Nutritional problems, overhydration and the association with quality of life in elderly dialysis patients. *International urology and nephrology,* 2012, vol. 44 (6), 1885-1892
- **S. SIPAHI ; E. HUR ; S. DEMIRTAS ; I. KOCAYIGIT ; D. BOZKURT ; A. TAMER ; H. GUNDUZ ; S. DUMAN.** Body composition monitor measurement technique for the detection of volume status in peritoneal dialysis patients: The effect of abdominal fullness. *International Urology and Nephrology,* 2011, vol. 43 (4), 1195-1199
- **L. LIU ; F. ZHU ; J. G RAIMANN ; S. THIJSSEN ; M. SIPAHIOGLU ; G. WYSTRYCHOWSKI ; T. KITZLER ; C. TETTA ; P. WABEL ; P. KOTANKO.** Determination of fluid status in haemodialysis patients with whole body and calf bioimpedance techniques. *Nephrology,* 2012, vol. 17 (2), 131-140
- **H.-R. CHUA ; L. XIANG ; P.-Y. CHOW ; H. XU ; L. SHEN ; E. LEE ; B.-W. TEO.** Quantifying acute changes in volume and nutritional status during haemodialysis using bioimpedance analysis. *Nephrology,* 2012, vol. 17 (8), 695-702
- **S. PARMENTIER ; H. SCHIRUTSCHKE ; B. SCHMITT ; J. SCHEWE ; K. HERBRIG ; F. PISTROSCH ; J. PASSAUER.** Influence of peritoneal dialysis solution on measurements of fluid status by bioimpedance spectroscopy. *International Urology and Nephrology,* 2013, vol. 45 (1), 229-232
- **S. POLONI ; I. SCHWEIGERT PERRY ; V. D'ALMEIDA ; I. SCHWARTZ.** Does phase angle correlate with hyperhomocysteinemia? a study of patients with classical homocystinuria. *Clinical Nutrition,* 2013, vol. 32 (3), 479-480
- **J. WILSON ; B. STRAUSS ; B. FAN ; F. DUEWER ; J. SHEPHERD.** Improved 4-compartment body-composition model for a clinically accessible measure of total body protein1-3. *American Journal of Clinical Nutrition,* 2013, vol. 97 (3), 497-504
- **F. SAMANI ; R. JABBARY ; O. MASHRABI.** Study on uterine artery blood flow in myomatous uterus. *Life Science Journal,* 2012, vol. 9 (4), 583-586
- **P. LUNDIN ; M. KARPEFORS ; K. CARLSSON ; M. HANSEN ; M. RUTH.** Bioimpedance spectroscopy: A new tool to assess early esophageal changes linked to gastroesophageal reflux disease?. *Diseases of the Esophagus,* 2011, vol. 24 (7), 462-469
- Comparison of transthoracic electrical bioimpedance cardiac output measurement with thermodilution method in post coronary artery bypass graft patients. **V. SHARMA ; A. SINGH ; B. KANSARA ; A. KARLEKAR.** Annals of Cardiac Anaesthesia. 2011, vol. 14, 104-110
- **A. SHARMA ; F. TIAN ; M. YIN ; M. KELLER ; M. COHEN ; P. TIEN.** Association of regional body composition with bone mineral density in HIV-infected and HIV-uninfected momen: Women's interagency HIV study. *Journal of Acquired Immune Deficiency Syndromes,* 2012, vol. 61 (4), 469-476

- **C. DI SOMMA ; L. ANGRISANI ; F. ROTA ; M. SAVANELLI ; T. CASCELLA ; A. BELFIORE ; F. ORIO ; G. LOMBARDI ; A. COLAO ; S. SAVASTANO.** Gh and igf-i deficiency are associated with reduced loss of fat mass after laparoscopic-adjustable silicone gastric banding. *Clinical Endocrinology,* 2008, vol. 69 (3), 393-399
- **E. SILLANPÄ ; S. CHENG ; K. HAKKINEN ; T. FINNI ; S. WALKER ; A. PESOLA ; J. AHTIAINEN ; L. STENROTH ; H. SELÄNNE ; S. SIPILÄ.** Body composition in 18- to 88-year-old adults-comparison of multifrequency bioimpedance and dual-energy x-ray absorptiometry. *Obesity,* 2014, vol. 22 (1), 101-109
- **L. DONINI ; E. POGGIOGALLE ; S. MIGLIACCIO ; A. AVERSA ; A. PINTO.** Body composition in sarcopenic obesity: systematic review of the literature. *Mediterranean Journal of Nutrition and Metabolism,* 2013, 1-8
- **P. MARIK.** Noninvasive cardiac output monitors: A state-of the-art review. *Journal of Cardiothoracic and Vascular Anesthesia,* 2013, vol. 27 (1), 121-134
- **M. SAVALLE ; F. GILLAIZEAU ; G. MARUANI ; E. PUYMIRAT ; F. BELLENFANT ; P. HOUILLIER ; J.-Y. FAGON ; C. FAISY.** Assessment of body cell mass at bedside in critically ill patients. *American Journal of Physiology - Endocrinology and Metabolism,* 2012, vol. 303 (3), 13
- **R. BAUMGARTNER ; S. HEYMSFIELD ; S. LICHTMAN ; J. WANG ; R. PIERSON JR.** Body composition in elderly people: Effect of criterion estimates on predictive equations. *American Journal of Clinical Nutrition,* 1991, vol. 53 (6), 1345-1353